# EUROPÄISCHE PATENTANMELDUNG

(11) **EP 2 110 116 A1**
(43) Veröffentlichungstag der Anmeldung: **21.10.2009**
(21) Anmeldenummer: 08007308.3
(22) Anmeldetag: 14.04.2008
(51) Int. Cl.: A61K 8/21, A61K 8/43, A61K 8/97, A61Q 11/00, A61Q 11/02

(54) **Wässrige antiseptische Zubereitung für den Mund- und Rachenraum**

(71) Anmelder: Tentan AG, 4452 Itingen (CH)
(72) Erfinder: Schaer, Michel, 4452 Itingen (CH)
(74) Vertreter: Schaad, Balass, Menzl & Partner AG

(57) **Zusammenfassung**

Wässrige antiseptische Zubereitung für den Mund- und Rachenraum, die Chlorhexidin und/oder ein Salz davon, pflanzliche Wirkstoffe und ein Alkali- und/oder Erdalkali-Fluorid enthält.

## Beschreibung

Die vorliegende Erfindung betrifft eine wässrige antiseptische Zubereitung für den Mund- und Rachenraum.

Für die Gesundheit des Menschen ist es von grosser Bedeutung, dass Mund- und Rachenraum gesund sind. Die regelmässige Reinigung und Pflege der Zähne, des Zahnfleischs und des Mund- und Rachenraums ist daher heutzutage ein wichtiges Bedürfnis und eigentlich eine Selbstverständlichkeit.

Oft werden ergänzend zur regelmässigen Reinigung antiseptische Zubereitungen, wie beispielsweise eine Mundspülung, eingesetzt, die den Mund- und Rachenraum zusätzlich desinfizieren und entzündungshemmend wirken. Dementsprechend gross ist die Auswahl an antiseptischen Zubereitungen für den Mund- und Rachenraum, die beispielsweise in Form von Zahnpasta, Mundwasser, Spüllösung oder Zahnpflege-Kaugummi erhältlich sind.

Die antiseptischen Zubereitungen für den Mund- und Rachenraum dienen in erster Linie der Desinfektion zur Verhinderung von Karies und Entzündungen, zum Beispiel von Paradontitis. Ausserdem enthalten die meisten solchen Zubereitungen olfaktorische Stoffe, wie beispielsweise Menthol oder Pfefferminzextrakt, die einen für den Benutzer angenehmen, frischen Geruch im Mund zurücklassen.

In letzter Zeit gewinnt der Einsatz von Implantaten zum Ersatz von einzelnen oder mehreren Zähnen immer mehr an Bedeutung. Der Einsatz eines Implantats erfolgt mittels eines chirurgischen Verfahrens. Dies führt dazu, dass im Mund- und Rachenraum eines Implantatträgers viel leichter Entzündungen entstehen, insbesondere im Bereich des Implantats, die zu schwerwiegenden Problemen und sogar zum Verlust des Implantats führen können. Deshalb ist es gerade für einen Implantatträger besonders wichtig, dass Mund- und Rachenraum optimal gereinigt und desinfiziert werden, und die Anforderungen an eine antiseptische Zubereitung für den Mund- und Rachenraum sind entsprechend gross.

In vielen im Handel erhältlichen antiseptischen Zubereitungen für den Mund- und Rachenraum ist Chlorhexidin, häufig in Form des wasserlöslichen Chlorhexiding-Guconats der Formel (I), als Wirkstoff enthalten. Chlorhexidin besitzt eine antiseptische Wirkung und dient der Desinfektion des Mund- und Rachenraums.

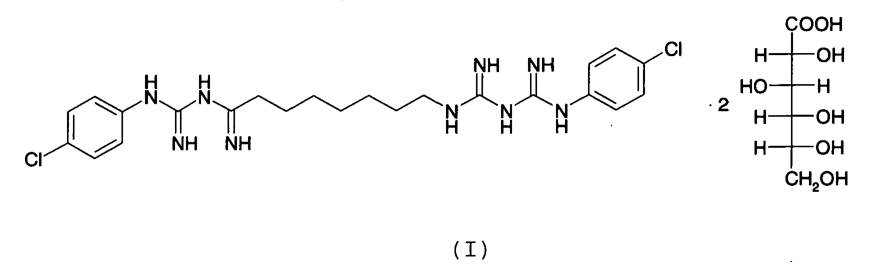

Ein gewichtiger Nachteil von Chlorhexidin ist, dass es zu bräunlichen Verfärbungen des Zahnschmelzes führt. Um diese unerwünschten Verfärbungen zu vermeiden, hat man versucht, den Chlorhexidingehalt von antiseptischen Zubereitungen für den Mund- und Rachenraum zu senken. Die Senkung des Chlorhexidingehalts führt aber dazu, dass die Wirkung dieser Zubereitungen abnimmt.

Ein weiterer Nachteil von Chlorhexidin liegt darin, dass es unangenehm schmeckt und nach der Anwendung einen unangenehmen Nachgeschmack hinterlässt. Dies führt dazu, dass Speisen und Getränke noch einige Zeit nach der Verwendung einer Chlorhexidin-haltigen antiseptischen Zubereitung schlecht schmecken.

Es ist daher eine Aufgabe der vorliegenden Erfindung, eine wirkungsvolle antiseptische Zubereitung für den Mund- und Rachenraum zur Verfügung zu stellen, die keinerlei Verfärbungen der Zähne verursacht, gut schmeckt und auch keinen unangenehmen Nachgeschmack im Mund hinterlässt.

Die Aufgabe wird gelöst durch eine wässrige Zubereitung gemäss Anspruch 1. Weitere bevorzugte Ausführungsformen sind in den abhängigen Ansprüchen beansprucht.

Die erfindungsgemässe wässrige Zubereitung für den Mund- und Rachenraum wirkt antiseptisch, besitzt also insbesondere desinfizierende und entzündungshemmende Eigenschaften. Die Zubereitung enthält Chlorhexidin und/oder ein Salz davon, pflanzliche Wirkstoffe sowie ein Alkali- und/oder Erdalkali-Fluorid. Als Chlorhexidin-Salz können beispielsweise das Chlorhexidin-Gluconat, das -Acetat und/oder das -Hydrochlorid eingesetzt werden. Zusätzlich kann die erfindungsgemässe Zubereitung natürlich die für Produkte dieser Art allgemein üblichen Zusatzstoffe, wie zum Beispiel Glycerin oder Ethanol, enthalten.

Der Begriff "pflanzliche Wirkstoffe", wie er hier verwendet wird, umfasst aus Pflanzen gewonnene Substanzen, die eine nützliche Wirkung aufweisen. Solche pflanzlichen Wirkstoffe können beispielsweise mittels Extraktion oder Destillation aus den Pflanzen oder einzelnen Bestandteilen davon gewonnen worden sein. Pflanzliche Wirkstoffe sind insbesondere desinfizierende, entzündungshemmende, antibakterielle, keimtötende, adstringierende, blutstillende, die Wundheilung fördernde und/oder schmerzstillende Substanzen aus Pflanzen.

Durch die Kombination von Chlorhexidin und/oder einem Salz davon mit pflanzlichen Wirkstoffen und einem Alkali- und/oder Erdalkali-Fluorid wird nicht nur eine optimale antiseptische Wirkung der erfindungsgemässen Zubereitung gewährleistet, sondern es werden auch die sonst üblichen Verfärbungen des Zahnschmelzes vermieden. Ausserdem hinterlässt die erfindungsgemässe Zubereitung keinen unangenehmen Nachgeschmack im Mund. Insbesondere für Träger von Zahnimplantaten kann so die gewünschte Desinfektion und Pflege des Mund- und Rachenraums garantiert werden, ohne dass dabei geschmackliche und ästhetische Nachteile in Kauf genommen werden müssten.

Die erfindungsgemässe wässrige Zubereitung für den Mund- und Rachenraum kann für die direkte Anwendung bestimmt sein. Bei dieser Ausführungsform ist es nicht nötig, die Zubereitung vor der Anwendung zu verdünnen. Beispiele für erfindungsgemässe Zubereitungen, die für die direkte Anwendung bestimmt sind, sind Spüllösungen oder Gels für den Mund- und Rachenraum.

Alternativ kann die erfindungsgemässe Zubereitung auch für die Anwendung in verdünnter Form bestimmt sein. Bei dieser Ausführungsform muss die Zubereitung vor der Anwendung verdünnt werden, beispielsweise mit Wasser. Ein Beispiel für eine solche Ausführungsform ist ein Mundwasser.

In einer bevorzugten Ausführungsform ist die erfindungsgemässe Zubereitung für die direkte Anwendung bestimmt und enthält Chlorhexidin und/oder ein Salz davon in einer Menge von weniger als 0.15 Gewichtsprozent, bevorzugt von weniger als 0.12 Gewichtsprozent, zum Beispiel in einer Menge von 0.11 oder 0.03 Gewichtsprozent (berechnet als Chlorhexidin).

In einer weiteren bevorzugten Ausführungsform ist die erfindungsgemässe Zubereitung für die Anwendung in verdünnter Form bestimmt und enthält Chlorhexidin und/oder ein Salz davon in einer Menge von 0.20-1.00 Gewichtsprozent, bevorzugt in einer Menge von 0.40-0.80 Gewichtsprozent, zum Beispiel in einer Menge von 0.55 Gewichtsprozent (berechnet als Chlorhexidin).

Vorzugsweise wird das Chlorhexidin in Form eines Chlorhexidin-Salzes, insbesondere als Chlorhexidin-Gluconat eingesetzt.

Als Alkali- und/oder Erdalkali-Fluorid enthält die erfindungsgemässe Zubereitung vorzugsweise Natrium-, Kalium- und/oder Calciumfluorid. Diese Fluorid-Salze sind gut wasserlöslich und eignen sich besonders gut für wässrige Präparate. Zudem sind diese Fluorid-Salze gut verträglich und erfüllen die Anforderungen für eine Kosmetikzulassung. Besonders bevorzugt ist Natriumfluorid.

Vorzugsweise enthält die erfindungsgemässe Zubereitung das Alkali- und/oder Erdalkali-Fluorid in einer Fluorid-Gesamtkonzentration von 0.02-0.20 Gewichtsprozent, insbesondere von 0.04-0.16 Gewichtsprozent, falls die Zubereitung für die direkte Anwendung bestimmt ist, und in einer Fluorid-Gesamtkonzentration von 0.002-0.010 Gewichtsprozent, insbesondere von 0.004-0.007 Gewichtsprozent, falls die Zubereitung für die Anwendung in verdünnter Form bestimmt ist.

Als pflanzliche Wirkstoffe enthält die erfindungsgemässe Zubereitung vorzugsweise Wirkstoffe aus Myrrhe, Salbei, Fenchel, Kamille, Nelke, Thymian, Pfefferminze und/oder Ratanhiawurzel.

Besonders bevorzugt ist die Kombination von pflanzlichen Wirkstoffen aus Myrrhe, Salbei und Pfefferminze. Salbei besitzt bekanntermassen adstringierende, antibakterielle, blutstillende und entzündungshemmende Eigenschaften, während Pfefferminze antibakteriell, entzündungswidrig, keimtötend und schmerzstillend wirkt. Zudem riecht Pfeffeminze angenehm frisch. Myrrhe wirkt desinfizierend, adstringierend und blutstillend und fördert die Narbenbildung.

In einer bevorzugten Ausführungsform liegt die erfindungsgemässe antiseptische Zubereitung in Form einer Flüssigkeit oder eines Gels vor. Diese Zubereitungsformen erlauben eine einfach, schnelle und unproblematische Anwendung der erfindungsgemässen Zubereitung im Mund- und Rachenraum. Eine flüssige Zubereitung kann beispielsweise als Mundwasser oder Spüllösung eingesetzt werden, ein Gel für zur Auftragung auf Zähnen und/oder Zahnfleisch.

Die vorliegende Erfindung soll anhand der nachfolgenden Beispiele weiter illustriert werden.

### Beispiel 1

Gel für den Mund- und Rachenraum mit einer Zusammensetzung gemäss einer der Varianten A, B, C oder D aus Tabelle 1. Das Gel ist für die direkte Anwendung bestimmt.

| **Tabelle 1** | | | | |
|---|---|---|---|---|
| | **A** | **B** | **C** | **D** |
| Chlorhexidingluconat-Lösung (20%) | 0.85 g | 1.00 g | 1.05 g | 1.25 g |
| Myrrhetinktur | 0.42 g | 0.38 g | 0.40 g | 0.30 g |
| Salbeiöl | 0.15 g | 0.10 g | 0.20 g | 0.10 g |
| Natriumfluorid | 0.20 g | 0.11 g | | |
| Calciumfluorid | | | 0.22 g | |
| Kaliumfluorid | | | | 0.15 g |
| Pfefferminzöl | 0.10 g | 0.16 g | 0.20 g | 0.12 g |
| Glycerin 1.23 | 5.00 g | 5.00 g | 5.00 g | 5.00 g |
| Xylitol | 9.50 g | 10.00 g | 10.10 g | 9.80 g |
| Ethanol (94%) | 15.00 g | 15.00 g | 15.00 g | 15.00 g |
| Natrosol 250 HHX-Pharma | 2.20 g | 2.20 g | 2.20 g | 2.20 g |
| Cremophor RH 40 | 2.50 g | 2.50 g | 2.50 g | 2.50 g |
| Wasser | 64.08 g | 63.43 g | 63.13 g | 63.53 g |

### Beispiel 2

Spüllösung für den Mund- und Rachenraum mit einer Zusammensetzung gemäss einer der Varianten E, F, G oder H aus Tabelle 2. Die Spüllösung ist für die direkte Anwendung bestimmt.

| **Tabelle 2** | | | | |
|---|---|---|---|---|
| | **E** | **F** | **G** | **H** |
| Chlorhexidingluconat-Lösung (20%) | 0.30 g | 0.25 g | 0.20 g | 1.25 g |
| Myrrhetinktur | 0.23 g | 0.19 g | 0.30 g | 0.20 g |
| Salbeiöl | 0.09 g | 0.05 g | 0.04 g | 0.12 g |
| Natriumfluorid | | 0.11 g | | |
| Calciumfluorid | 0.32 g | | 0.22 g | |
| Kaliumfluorid | | | | 0.14 g |
| Pfefferminzöl | 0.10 g | 0.08 g | 0.05 g | 0.11 g |
| Glycerin 1.23 | 2.80 g | 3.00 g | 2.90 g | 3.10 g |
| Xylitol | 2.95 g | 3.00 g | 3.20 g | 2.80 g |
| Ethanol (94%) | 13.50 g | 13.50 g | 13.50 g | 13.50 g |
| Acesulfam-K | 0.05 g | 0.03 g | 0.03 g | 0.02 g |
| Cremophor RH 40 | 1.50 g | 1.50 g | 1.50 g | 1.55 g |
| Wasser | 78.48 g | 77.77 g | 78.06 g | 77.22 g |

### Beispiel 3

Mundwasser für den Mund- und Rachenraum mit einer Zusammensetzung gemäss einer der Varianten I, J, K oder L aus Tabelle 3. Das Mundwasser ist für die Anwendung in verdünnter Form bestimmt.

| **Tabelle 3** | | | | |
|---|---|---|---|---|
| | **I** | **J** | **K** | **L** |
| Chlorhexidingluconat-Lösung (20%) | 60 mg | 50 mg | 45 mg | 55 mg |
| Myrrhetinktur | 42 mg | 38 mg | 40 mg | 37 mg |
| Salbeiöl | 5 mg | 10 mg | 11 mg | 8 mg |
| Natriumfluorid | 0.15 mg | 0.11 mg | | 0.10 mg |
| Calciumfluorid | | | 0.10 mg | |
| Pfefferminzöl | 12 mg | 15 mg | 16 mg | 14 mg |
| Glycerin 1.23 | 254 mg | 267 mg | 259 mg | 270 mg |
| Ethanol (94%) | 627 mg | 620 mg | 629 mg | 616 mg |

## Patentansprüche

1. Wässrige antiseptische Zubereitung für den Mund- und Rachenraum, enthaltend Chlorhexidin und/oder ein Salz davon, pflanzliche Wirkstoffe und ein Alkali- und/oder Erdalkali-Fluorid.

2. Zubereitung gemäss Anspruch 1, **dadurch gekennzeichnet, dass** sie für die direkte Anwendung bestimmt ist.

3. Zubereitung gemäss Anspruch 1, **dadurch gekennzeichnet, dass** sie für die Anwendung in verdünnter Form bestimmt ist.

4. Zubereitung gemäss Anspruch 2, **dadurch gekennzeichnet, dass** sie Chlorhexidin und/oder ein Salz davon in einer Menge von weniger als 0.15 Gewichtsprozent, insbesondere in einer Menge von weniger als 0.12 Gewichtsprozent, enthält.

5. Zubereitung gemäss Anspruch 3, **dadurch gekennzeichnet, dass** sie Chlorhexidin und/oder ein Salz davon in einer Menge von 0.20-1.00 Gewichtsprozent, insbesondere in einer Menge von 0.40-0.80 Gewichtsprozent, enthält.

6. Zubereitung gemäss einem der vorhergehenden Ansprüche, **dadurch gekennzeichnet, dass** sie als Alkali- und/oder Erdalkali-Fluorid Natrium-, Kalium- und/oder Calciumfluorid enthält.

7. Zubereitung gemäss Anspruch 6, **dadurch gekennzeichnet, dass** sie als Alkali-Fluorid Natriumfluorid enthält.

8. Zubereitung gemäss Anspruch 2, **dadurch gekennzeichnet, dass** sie das Alkali- und/oder Erdalkali-Fluorid in einer Fluorid-Gesamtkonzentration von 0.02-0.20 Gewichtsprozent enthält.

9. Zubereitung gemäss Anspruch 3, **dadurch gekennzeichnet, dass** sie das Alkali- und/oder Erdalkali-Fluorid in einer Fluorid-Gesamtkonzentration von 0.002-0.010 Gewichtsprozent enthält.

10. Zubereitung gemäss einem der vorhergehenden Ansprüche, **dadurch gekennzeichnet, dass** sie pflanzliche Wirkstoffe aus Myrrhe, Salbei, Fenchel, Kamille, Nelke, Thymian Pfefferminze und/oder Ratanhiawurzel enthält.

11. Zubereitung gemäss einem der vorhergehenden Ansprüche, **dadurch gekennzeichnet, dass** sie pflanzliche Wirkstoffe aus Myrrhe, Salbei und Pfefferminze enthält.

12. Zubereitung gemäss einem der vorhergehenden Ansprüche, **dadurch gekennzeichnet, dass** sie in Form einer Flüssigkeit oder eines Gels vorliegt.
